# EUROPEAN PATENT APPLICATION

(11) **EP 4 039 735 A1**
(43) Date of publication of application: **10.08.2022**
(21) Application number: 20872771.9
(22) Date of filing: 01.10.2020
(51) Int. Cl.: C08J 5/00, A61L 2/02, B24C 1/00

(54) **ANTIBACTERIAL MOLDED ARTICLE AND METHOD FOR MANUFACTURING SAME**

(30) Priority: 04.10.2019 JP 2019184043
(71) Applicant: Kureha Corporation, Chuo-ku Tokyo 103-8552 (JP); Japan Aerospace Exploration Agency, Tokyo 182-8522 (JP); Fuji Manufacturing Co., Ltd., Tokyo 132-0025 (JP)
(72) Inventor: ABE, Shunsuke, Tokyo 103-8552 (JP); SHIMIZU, Kazuhiko, Tokyo 103-8552 (JP); ONIZAWA, Kayoko, Tokyo 103-8552 (JP); YAMAZAKI, Masahiro, Tokyo 103-8552 (JP); GOTO, Aki, Chofu-shi, Tokyo 182-8522 (JP); YANAGASE, Keiichi, Chofu-shi, Tokyo 182-8522 (JP); MIYAZAKI, Eiji, Chofu-shi, Tokyo 182-8522 (JP); UCHIUMI, Yusuke, Tokyo 132-0025 (JP); FUKUI, Eri, Tokyo 132-0025 (JP)
(74) Representative: Hoefer & Partner Patentanwälte mbB
(86) International application number: PCT/JP2020/037459
(87) International publication number: WO 2021/066107

(57) **Abstract**

The present invention is to provide an antibacterial molded article that can increase options for the material and shape of the molded article, and a method for manufacturing the same. The object is achieved by an antibacterial molded article containing a plastic molded article, the plastic molded article having a rough region having an arithmetic average roughness Ra of a roughness curve in accordance with JIS B 0601 (2013) of 0.14 µm or greater and 0.72 µm or less provided on a surface with which bacteria can come into contact.

## Description

### TECHNICAL FIELD

The present invention relates to an antibacterial molded article and a method for manufacturing the same.

### BACKGROUND ART

Antibacterial articles are increasingly on the market due to increase in consumer awareness. Many antibacterial articles are those obtained by applying coating containing an antibacterial agent or obtained by embedding silver nanoparticles; however, the former may lose its antibacterial activity over time, and the latter may have too high antibacterial activity and there are restrictions on the use environment due to safety of a living body.

In recent years, patent application and thesis submission have been made on technologies that exhibit antibacterial action by physical needling by forming a nano-order surface protrusion and recess structure on a surface. For example, Patent Documents 1 and 2 describe methods of imparting antibacterial action to a surface of a resin composition by forming a plurality of nano-sized protrusions on the surface. According to the methods described in these documents, it is presumed that the antibacterial action is exhibited by piercing and killing bacteria, which are brought into contact with the protrusion, by the protrusion. With this mechanism, high antibacterial effect can be expected to multidrug-resistant bacteria that are problematic recently.

Specifically, Patent Document 1 describes an antibacterial article exhibiting antibacterial performances by facilitating contact with cells by making the spacing between protrusions adequately smaller than the size of bacteria and making the shape of the protrusion a needle shape with a large aspect ratio that can pierce bacteria. Note that Patent Document 1 describes that the antibacterial action is enhanced when the static contact angle of pure water on the surface of the antibacterial article is 30° or less because the surface becomes hydrophilic and the bacteria tends to be pierced by microspikes. According to Patent Document 1, the antibacterial article can be produced by a method, in which a liquid resin composition is cured while an original plate having a desired protrusion and recess shape is pressed toward a surface of the liquid resin composition.

Furthermore, Patent Document 2 describes a synthetic polymer film having a plurality of raised portions, a two-dimensional size of the plurality of raised portions is in a range of more than 20 nm and less than 500 nm when viewed in a normal direction of the synthetic polymer film. Note that Patent Document 2 describes that, when a contact angle of hexadecane on a surface of the synthetic polymer film is 51° or less, bactericidal activity is suitable but a contact angle of water (hydrophilicity) is not directly related to the bactericidal action. According to Patent Document 2, the synthetic polymer film can be produced by a method, in which a UV curable resin is cured while an anodized porous alumina layer as a mold is pressed toward a surface of the UV curable resin.

Furthermore, Patent Document 3 describes an antibacterial and antifungal article having a plurality of microspikes arranged, in which an average distance between microspikes is 1 µm or less, a height of the microspike is 80 nm or greater and 1000 nm or less, and the microspike has a shape in which a shape around a tip of the microspike is thinner than that of a bottom part. According to Patent Document 3, because sizes of bacteria are generally 1 µm, by setting the shape and arrangement of the microspikes as described above, bacteria and fungi do not enter between the microspikes but are brought into contact with the tip of the microspike and pierced by the tip of the microspike and thus killed.

Furthermore, Patent Document 4 describes an antibacterial article having a plurality of microspikes arranged, in which an average distance between microspikes having a height of 0.125 µm or greater is greater than 0.5 µm and 5.0 µm or less. According to Patent Document 4, by setting the average of the distance between microspikes to greater than 0.5 µm and 5.0 µm or less, it is described that, bacteria can be killed by attaching bacteria and piercing bacteria by the microspikes or the like, and production is easy.

### CITATION LIST

### Patent Document

Patent Document 1: JP 2016-093939 A
Patent Document 2: JP 2016-120478 A
Patent Document 3: JP 2016-215622 A
Patent Document 4: JP 2017-132916 A

### SUMMARY OF INVENTION

### Technical Problem

As described in Patent Documents 1 to 4, compared to the case of a resin molded article obtained by applying an antibacterial agent on a surface of a molded article, a resin molded article obtained by forming a plurality of nano-sized protrusions on a surface of a plastic molded article is less likely to cause reduction in antibacterial action caused by peeling or shedding of an antibacterial agent, and thus it is expected that the antibacterial performance can be maintained for a longer period of time.

Meanwhile, in Patent Documents 1 to 4, the microspikes are formed by curing a resin composition while a protrusion and recess face of an original plate having a desired protrusion and recess shape is pressed toward a coated film of the resin composition. However, by this method, protrusions having antibacterial action are only formed on a curable resin, and there are problems of limitations on applicable raw materials and low productivity.

The present invention is completed in light of the problems described above, and an object of the present invention is to provide an antibacterial molded article that can increase options for the material and shape of the molded article, and a method for manufacturing the same.

### SOLUTION TO PROBLEM

An antibacterial molded article of an aspect of the present invention to solve the problems described above includes a plastic molded article, the plastic molded article having a rough region having an arithmetic average roughness Ra of a roughness curve in accordance with JIS B 0601 (2013) of 0.14 µm or greater and 0.72 µm or less provided on a surface with which bacteria can come into contact.

A method for manufacturing an antibacterial molded article related to another aspect of the present invention to solve the problems described above includes: preparing a base material containing a plastic on a surface, and performing a blast treatment at least on the surface containing the plastic of the base material.

### ADVANTAGEOUS EFFECTS OF INVENTION

According to the present invention, an antibacterial molded article that can increase options for the material and shape of the molded article, and a method for manufacturing the same are provided.

### BRIEF DESCRIPTION OF DRAWINGS

FIG. 1A is a photograph of a surface of Sample 1-1 in Experiment 1 taken by a scanning electron microscope (SEM). FIG. 1B is a photograph of a cross section of Sample 1-1 taken by an SEM.
FIG. 2A is a photograph of a surface of Sample 2-1 (LLDPE) in Experiment 2 taken by a scanning electron microscope (SEM). FIG. 2B is a photograph of a cross section of Sample 2-1 (LLDPE) taken by an SEM.
FIG. 3A is a photograph of a surface of Sample 2-3 (PVDF) in Experiment 2 taken by a scanning electron microscope (SEM). FIG. 3B is a photograph of a cross section of Sample 2-3 (PVDF) taken by an SEM.
FIG. 4A is a photograph of a surface of Sample 2-4 (PVDC) in Experiment 2 taken by a scanning electron microscope (SEM). FIG. 4B is a photograph of a cross section of Sample 2-4 (PVDC) taken by an SEM.
FIG. 5A is a photograph of a surface of Sample 2-5 (PPS) in Experiment 2 taken by a scanning electron microscope (SEM). FIG. 5B is a photograph of a cross section of Sample 2-5 (PPS) taken by an SEM.
FIG. 6A is a photograph of a surface of Sample 2-6 (PET) in Experiment 2 taken by a scanning electron microscope (SEM). FIG. 6B is a photograph of a cross section of Sample 2-6 (PET) taken by an SEM.

### DESCRIPTION OF EMBODIMENTS

As a result of diligent study on the problems described above, the present inventors found that adequate antibacterial action can be exhibited by using a plastic molded article having a rough region having an arithmetic average roughness Ra of 0.14 µm or greater and 0.72 µm or less provided on a surface with which bacteria can come into contact, and conducted further study to complete the present invention.

### Antibacterial Molded Article

A first embodiment of the present invention relates to an antibacterial molded article including a plastic molded article, and the plastic molded article has a rough region having an arithmetic average roughness Ra of 0.14 µm or greater and 0.72 µm or less provided on a surface.

The antibacterial molded article is only required to have the plastic molded article having the rough region on a surface, and the entirety thereof may be formed from the plastic molded article, or the antibacterial molded article may be a complex of the plastic molded article and another plastic molded article that does not have the rough region on a surface or may be a complex of the plastic molded article and a metal or ceramic. Note that "antibacterial molded article" is a molded article provided with an antibacterial action by the rough region, and the antibacterial molded article itself may be used for antibacterial purpose, or the antibacterial molded article may be a molded article provided with an antibacterial action in addition to its primary purpose of the antibacterial molded article.

The plastic molded article is a molded article obtained by forming a plastic into a predetermined shape or indeterminate shape and provided with a shape. The shape of the plastic molded article is not particularly limited and can be optionally selected based on the purpose of the antibacterial molded article. For example, the molded article can have a predetermined shape such as a film shape, a sheet shape, a tube shape, a ring shape, a bulk shape (e.g., cube, cuboid, column, and sphere), a plate shape, a bag shape, a fiber shape, a net-like shape, and a three dimensional structure obtained by processing these, or can have an indeterminate shape.

For example, when the shape is a film shape or sheet shape, the thickness of the plastic molded article is preferably 1 µm or greater and 1000 µm or less, more preferably 3 µm or greater and 800 µm or less, even more preferably 5 µm or greater and 500 µm or less, and particularly preferably 10 µm or greater and 300 µm or less.

The present invention has advantages in that mass production is possible by, for example, injection molding by forming the rough region on a surface of the plastic molded article, high degree of freedom in design is achieved and processing into various shapes is possible, flexibility is achieved and adhesion to various shapes is possible, and weight reduction of the antibacterial molded article can be performed compared to a case of a metal.

The rough region needs to be at least a part of the surface of the plastic molded article, but the entirety of the surface of the plastic molded article may be the rough region. Furthermore, in the plastic molded article having a plurality of surfaces, such as a polyhedron, the rough region may be formed on all of the plurality of surfaces or may be formed on at least one surface (entirety of the surface or a part of the area included in the surface). Alternatively, in the plastic molded article having at least one surface, such as a sphere, the rough region may be formed on the entirety of the one surface or a part of the area included in the one surface.

The rough region is formed on a surface, with which bacteria can come into contact, of the antibacterial molded article. The surface with which bacteria can come into contact means a surface of an antibacterial molded article, the surface having a possibility of being in contact with bacteria from the outside during use, standby, and storage of the antibacterial molded article. For example, the rough region may be formed on a portion with which a user is in contact at the time of use of the antibacterial molded article. Furthermore, when there is a portion expected to have more opportunities of contact with bacteria, such as a portion subjected to water flow or air flow at the time of use, or a portion expected to be in contact with food, pharmaceuticals, or living bodies, the rough region may be formed at least on the corresponding area. When the plastic molded article has a shape such as a bag shape or a tube shape, the rough region may be formed in the inner surface thereof. Furthermore, the rough region does not need to be exposed to outside when not used, for example, by sealing treatment or a cap.

The rough region has an arithmetic average roughness Ra of 0.14 µm or greater and 0.72 µm or less. When the Ra is 0.14 µm or greater, it is conceived that the protrusion and recess shape with an adequate height (depth) is formed on the rough region, bacteria are captured in the recess part of the protrusion and recess shape, and free motion and proliferation of the bacteria can be suppressed, and thus adequate antibacterial action by the rough region can be achieved. On the other hand, when the Ra is 0.72 µm or less, it is conceived that, because the height of the protrusion and recess formed in the rough region is not too high, bacteria can be captured by both the protrusion part and the recess part (by both oblique side and bottom part included in the protrusion and recess shape), and the area capable of capturing bacteria can be made larger, and thus free motion and proliferation of the bacteria can be more effectively suppressed. Furthermore, the protrusion part is less likely to be worn at the time of use, so that the antibacterial effect can be easily maintained. The appearance of the molded article is also good. From the perspective described above, the Ra is preferably 0.14 µm or greater and 0.70 µm or less, more preferably 0.20 µm or greater and 0.60 µm or less, even more preferably 0.23 µm or greater and 0.50 µm or less, and particularly preferably 0.25 µm or greater and 0.35 µm or less.

Furthermore, the rough region preferably has an average length Rsm of a profile element of 1.0 µm or greater and 50.0 µm or less. When the Rsm is 1.0 µm or greater, the length of a recess part between a protrusion part and a neighboring protrusion part is greater than the size of one bacterium, adequate antibacterial action can be achieved by the rough region. The mechanism of antibacterial action exhibition is not clear; however, it is conceived that bacteria are captured in the recess part, the motion thereof is suppressed, and thus proliferation is suppressed. For example, the size of *Escherichia coli* is 0.5 µm × 1.0 to 2.0 µm, and the size of *Staphylococcus aureus* is 0.5 µm × 1.0 µm. From the perspective of ensuring suppression of motion of bacteria, Rsm is preferably 5.0 µm or greater. Meanwhile, when the Rsm is 50.0 µm or less, because the rough region has an adequate number of recess parts in a unit length (unit area), adequate antibacterial action by the rough region can be achieved. From the perspective described above, the Rsm is more preferably 10.0 µm or greater and 33.0 µm or less, even more preferably 13.0 µm or greater and 32.0 µm or less, and particularly preferably 15.0 µm or greater and 30.0 µm or less.

The rough region preferably has a large number of microscopic concavities. It is conceived that, in the rough region having the arithmetic average roughness Ra of 0.14 µm or greater and 0.72 µm or less and having a large number of microscopic concavities, the size of the concavity is roughly identical to the size of bacterium. Thus, it is conceived that the concavities can capture bacteria, which have come into contact with the rough region, in the concavities. It is conceived that the concavities can suppress division of the bacteria, and thus proliferation of the bacteria is suppressed, and the number of the bacteria is reduced. For example, the rough region can be formed regardless of the resin type by changing the conditions of blast treatment described below depending on the physical properties of the resin type.

Furthermore, the ratio of the Ra to the Rsm (Ra/Rsm) is preferably 0.004 or greater and 0.720 or less. When the Ra/Rsm is 0.004 or greater and 0.720 or less, it is conceived that, because the slope of each concavity becomes an adequate slope, bacteria can be captured by both the protrusion part and the recess part (by both oblique side and bottom part included in the protrusion and recess shape), and the area capable of capturing bacteria can be made larger, and thus free motion and proliferation of the bacteria can be more effectively suppressed. From the perspective described above, the Ra/Rsm is more preferably 0.005 or greater and 0.500 or less, more preferably 0.007 or greater and 0.100 or less, particularly preferably 0.008 or greater and 0.050 or less, particularly preferably 0.009 or greater and 0.030 or less, and particularly preferably 0.010 or greater and 0.025 or less.

Note that, in the present specification, the arithmetic average roughness Ra and the average length Rsm of the profile element are parameters determined with reference to JIS B 0601 (2013). Specifically, first, an image of a cross section of the base material is taken by a 3D Laser Scanning Microscope (Vk-X250). The obtained image is processed to identify open traverse tracing the surface. Then, based on the open traverse, slopes and undulations are removed by an ordinary method, and a roughness curve is obtained. For 10 µm portion of a freely-chosen position in the roughness curve, an average value of absolute values is taken as the arithmetic average roughness Ra, and an average value of lengths of profile element is taken as the average length Rsm of the profile element. The Ra and Rsm are values measured by using a 3D Laser Scanning Microscope (Vk-X250) available from Keyence Corporation.

Furthermore, in the present specification, the height of the microspike can be a value measured by the following method. First, the non-closed chain side of the open traverse of the analysis result of the cross section of the base material by the laser microscope is taken as a bottom part of a protrusion, and the open traverse side relative to the bottom part is taken as a protrusion. In a triangle formed by connecting freely chosen one vertex of the open traverse and freely chosen two vertexes on the open traverse in the bottom part side for each protrusion, the height of a triangle having the largest height from the side connecting two vertexes of the bottom part side (referred to as bottom side) is taken as a height of each protrusion.

The rough region has antibacterial action against various bacteria. For example, the rough region has antibacterial action for at least one of *Escherichia coli, Staphylococcus aureus,* lactic acid bacteria, or *Pseudomonas aeruginosa,* preferably has antibacterial action for at least one of *Escherichia coli, Staphylococcus aureus,* or *Pseudomonas aeruginosa,* and preferably has antibacterial action for at least *Escherichia coli* and *Staphylococcus aureus.*

Note that, in the present specification, antibacterial action refers to both killing bacteria present in a medium and suppressing proliferation of bacteria by inactivating the bacteria. Furthermore, in the present specification, exhibiting antibacterial action means that Δlog bacterial count determined by inoculating bacteria and measuring a viable bacteria count by a method identical to the method described in JIS Z 2801 (2012) immediately after the inoculation and at 24 hours after the inoculation (logarithmic value of viable bacteria count at 24 hours after unprocessed sample - logarithmic value of viable bacteria count at 24 hours after evaluation sample having a surface having the rough region) is 0.2 or greater. Note that Δlog is more preferably 1.0 or greater, even more preferably 2.0 or greater, yet even more preferably 3.0 or greater, and particularly preferably 4.0 or greater.

The plastic molded article may be a molded article formed by molding a composition containing a resin (resin composition). The resin composition contains 30 mass% or greater of the resin with respect to the total mass thereof. The resin composition may optionally contain an additive to adjust the characteristics of the antibacterial molded article based on, for example, the purpose of the antibacterial molded article. Examples of the additive include known fillers (fillers), lubricants, plasticizers, UV stabilizers, coloration inhibitors, matting agents, deodorizing agents, flame retarders, weathering agents, antistatic materials, antioxidants, and colorants (dyes, pigments). These additives may be used by selecting an optimal combination in a range that does not inhibit the effect of the present invention. Furthermore, depending on the purpose or request, organic substances (may be another polymer) and inorganic substances such as metal nanoparticles may be used as other additives.

The type of the resin can be optionally selected based on the purpose of the antibacterial molded article. The resin may be a thermoplastic resin or a curable resin. The curable resin may be a thermosetting resin, a photocurable resin, or an electron beam curable resin. Furthermore, the resin may be a crystalline resin or an amorphous resin. Furthermore, the resin may be a rubber such as a synthetic rubber or a natural rubber. Note that, according to the knowledge of the present inventors, a plastic molded article containing a crystalline resin is easily processed and, for example, the conditions of blast treatment described below is moderate compared to a plastic molded article containing an amorphous resin, and thus antibacterial action is easily imparted. However, even in a case of a plastic molded article containing a resin other than a crystalline resin, antibacterial action can be adequately imparted by appropriately adjusting, for example, the blast treatment conditions.

Examples of the resin include polyethylene (e.g., including linear low density polyethylene (LLDPE), low density polyethylene (LDPE), and high density polyethylene (HDPE)), polypropylene (e.g., including unstretched polypropylene (CPP) and stretched polypropylene (OPP) such as uniaxially stretched polypropylene and biaxially stretched polypropylene), other polyolefin-based resins, polytetrafluoroethylene (PTFE), perfluoroalkoxy alkane (PFA), perfluoroethylene propene copolymers (FEP), polyvinylidene fluoride (PVDF), polyvinylidene chloride (PVDC), non-aromatic polyamide (e.g., including nylon 6, nylon 66, and nylon 12), non-aromatic polyimide, polyacetal (POM), polyurethane, ethylene-vinyl alcohol copolymers (EVOH), polyvinyl chloride (PVC), acrylic polymers, ethylene-vinyl acetate copolymers (EVA), polylactic acid (PLA), polycaprolactone (PCL), and polyglycolic acid (PGA), polystyrene (PS), polyester (e.g., including polyethylene terephthalate (PET), polybutylene terephthalate (PBT), and polybutylene naphthalate (PBN)), polyphenylene sulfide (PPS), polyether ether ketone (PEEK), semiaromatic polyamide (e.g., including nylon 6T and nylon 9T), wholly aromatic polyamide, semiaromatic polyimide, wholly aromatic polyimide, polystyrene (PS), acrylonitrile-styrene copolymers (AS), acrylonitrile-butadiene-styrene copolymers (ABS), polycarbonate (PC), polyarylate (PAR), polyphenylene ether (PPE), polyphenol-based resins, and epoxy resins.

Examples of the synthetic rubber include isoprene rubber (IR), butadiene rubber (BR), styrene-butadiene rubber (SBR), nitrile rubber (NBR), ethylene-propylene rubber (EPM), and other thermoplastic elastomers (including SEBS, SBS, and SEPS).

Among these resins, polyolefin-based resins, polytetrafluoroethylene (PTFE), perfluoroalkoxy alkane (PFA), perfluoroethylene propene copolymers (FEP), polyvinylidene fluoride (PVDF), polyvinylidene chloride (PVDC), polyamide (PA), polyimide (PI), polyphenylene sulfide (PPS), polyether ether ketone (PEEK), polyacetal (POM), acrylonitrile-butadiene-styrene copolymers (ABS) resins, polycarbonate (PC), polyethylene terephthalate (PET), polyurethane, ethylene-vinyl alcohol copolymers (EVOH), polyvinyl chloride (PVC), acrylic polymers, ethylene-vinyl acetate copolymers (EVA), polylactic acid (PLA), polycaprolactone (PCL), and polyglycolic acid (PGA) are preferred, polyethylene, polypropylene, polyvinylidene chloride, polyethylene terephthalate, and polyphenylene sulfide are more preferred, polyethylene, polypropylene, polyvinylidene chloride, and polyphenylene sulfide are more preferred, polyethylene, polypropylene, and polyvinylidene chloride are even more preferred, polyethylene is particularly preferred, and LLDPE is particularly preferred.

The antibacterial molded article can be used for a wide variety of purposes including a packaging, equipment for a building, a home appliance, an electronic device or peripheral device thereof, a component for an automobile, various coatings, medical equipment, an article for agriculture, stationery, and body outfit. Note that an aspect of use of the antibacterial molded article include articles, in which the antibacterial molded article is used for these purposes, and incorporating the antibacterial molded article as a part of these articles.

For example, when the antibacterial molded article is used for packaging purpose described above, storability of an article to be packaged can be enhanced by forming the rough region in an outer surface or internal surface of a film or sheet to be used for packaging the article and preventing entrance of bacteria to the packaged article.

Examples of the equipment for a building include toilets and sheets for toilet seats, washstands, pipes for plumbing, foot wiping mats, interior finishing materials, and articles to which human hands ordinarily touch, such as doorknobs for doors, handrails, and switches.

Examples of the home appliance include rice cookers, microwave ovens, refrigerators, irons, hairdryers, air conditioners, and air purifiers.

Examples of the electronic device or peripheral device thereof include laptop personal computers, smartphones, tablets, digital cameras, electronic devices for medical treatment, POS systems, printers, televisions, mouses, and keyboards.

Examples of the component for an automobile include steering wheels, seats, shift levers, and various pipes.

Examples of the packaging include packaging for pharmaceuticals and foods.

Examples of the coating include coatings for wall surfaces, floor surfaces, and ceilings of factories, operating rooms, storages, and container for transport.

Examples of the medical equipment include forceps, syringes, stents, artificial blood vessels, catheters, wound dressings, scaffoldings for regenerative therapy, antiadhesive materials, or pacemakers.

Examples of the article for agriculture include spread films for greenhouses.

Examples of the body outfit include clothing including outerwear and underwear, headgear, shoes, gloves, diapers, and sanitary napkins and storage bags thereof.

Furthermore, the antibacterial molded article can be used to sterilize bacteria contained in equipment for buildings, body outfits, tableware, beverages, and foods by bringing the antibacterial molded article into contact with these.

### Method for Manufacturing Antibacterial Molded Article

The antibacterial molded article can be manufactured by a method including mechanically processing a surface containing a plastic of a molded article base material containing the plastic on the surface (hereinafter, also simply referred to as "base material") and forming the surface into a rough region having the shape described above.

Specifically, first, a base material, in which a rough region is to be formed on a surface, is prepared. The material and shape of the plastic contained in the surface of the base material are as described above for the plastic molded article.

The mechanical processing is performed for a region in which the rough region is to be formed in the plastic of the surface (region in which antibacterial action is to be provided) among surfaces of the base material. The mechanical processing is not particularly limited as long as the shape described above can be provided on the surface and, for example, the surface of the base material may be abraded by blast treatment and abrasion with sandpaper and a grinder. By the methods described in Patent Documents 1 to 4, protrusions having antibacterial action are only formed on a curable resin, applicable raw materials are limited and the shape of the molded article, to which antibacterial action can be provided, is almost limited to a film shape. However, by forming the rough surface by these methods, a plastic molded article having the rough surface can be manufactured regardless of the material or shape. As an example of these mechanical processing, an example of blast treatment will be described below.

The blast treatment may be performed by a known method by which an abrasive media energized by a compressed fluid is allowed to collide with the surface containing the plastic of the base material. At this time, the conditions of the blast treatment are only required to be changed based on, for example, the physical properties of the resin composition to form the rough region. Note that the abrasive media is only required to collide with at least the surface containing the plastic among surfaces of the base material, but some of the abrasive media may collide with a surface containing a material other than the plastic.

The compressed fluid may be a gas such as the air, may be a liquid such as water, and may be a mixed fluid of gases and liquids.

The blasting pressure (processing pressure) of the compressed fluid may be selected in the range from, for example, 0.01 MPa to 5 MPa. The processing pressure is preferably 0.03 MPa or greater and 0.7 MPa or less, and more preferably 0.05 MPa or greater and 0.5 MPa or less.

The abrasive media may be selected from abrasive grains formed from a known material, including plants (e.g., seed fragments), metals (e.g., steel and stainless steel), ceramics (e.g., fused alumina, silicon carbide, and zircon), plastics (e.g., polyamide and melamine resins), solids that sublimate such as dry ice, and other inorganic materials such as diamond and glass.

In the present embodiment, the abrasive media is preferably particles containing the abrasive grains described above and elastic bodies. The elastic bodies absorb shock when the abrasive media collide with the surface of the base material, and facilitates formation of the rough region having the shape described above. For example, the abrasive media can be a particle in which the abrasive grains are dispersed inside or on the surface of an elastic body particle that serves as a base material, or a particle in which the abrasive grains are adhered or attached to the surface of a resin particle that serves as a base material. As the abrasive media, typically, those having a particle size from 20 to 20000 mesh and preferably from 100 to 10000 mesh are used.

The elastic body as the base material is preferably an elastic body having elasticity and having a relatively small rebound resilience, and can be various rubbers, thermoplastic elastomers, and gels.

The rubber may be a natural rubber or a synthetic rubber. Examples of the synthetic rubber include isoprene rubber, styrene-butadiene rubber, butadiene rubber, acrylonitrile-butadiene rubber, chloroprene rubber, ethylene propylene rubber, chlorosulfonated polyethylene, chlorinated polyethylene, urethane rubber, silicone rubber, epichlorohydrin rubber, and butyl rubber.

Examples of the thermoplastic elastomer include styrene block copolymers, chlorinated polyethylene-based elastomers, polyester-based elastomers, nitrile-based elastomers, fluorine-based elastomers, silicon-based elastomers, halogen ester-based polymer alloys, olefin-based elastomers, vinyl chloride-based elastomers, urethane-based elastomers, polyamide-based elastomers, and halogen ester-based polymer alloys.

Examples of the gel include mixtures of water and gelatin.

The abrasive media is preferably Sirius Media SIG080-7, available from Fuji Manufacturing Co., Ltd. The Sirius Media SIG080-7 is an abrasive media obtained by adhering and assembling 8000 mesh abrasive grains of green silicon carbide by synthetic rubber elastic bodies. Blast processing with an ordinary single abrasive grain, such as SiC, forms simple surface roughness by a single vector collision. On the other hand, SIG080-7 can form a target surface by a phenomenon involving a complex abrasion caused by addition of a vector in the transverse direction on the surface due to deformation at the time of collision caused by the effect of the elastic bodies.

The spray rate of the abrasive media may be selected in the range from, for example, 0.1 kg/min to 10 kg/min. The spray rate is preferably 0.3 kg/min or greater and 9 kg/min or less, and more preferably 0.5 kg/min or greater and 8 kg/min or less.

The angle of blast stream of the abrasive media is preferably adjusted in a manner that the abrasive media collide with the surface of the base material from a direction close to perpendicular. The angle of blast stream can be 20 degrees or greater and 160 degrees or less, and is preferably 25 degrees or greater and 165 degrees or less, and more preferably 30 degrees or greater and 150 degrees or less, with respect to the surface of the base material.

The distance of spraying of the abrasive media can be, for example, 10 mm or greater and 700 mm or less, and preferably 20 mm or greater and 600 mm or less, and more preferably 30 mm or greater and 500 mm or less, from the blast nozzle to the material to be blasted.

The processing time of the blast treatment may be selected in a range from, for example, 0.1 sec/cm² to 20 sec/cm². The processing time is preferably 0.5 sec/cm² or greater and 15 sec/cm² or less, and more preferably 0.1 sec/cm² or greater and 10 sec/cm² or less.

In the present embodiment, the surface of the base material is preferably cooled at the time of the blast treatment. When heat build-up occurs in the base material due to collision of the abrasive media, the plastic on the surface which has been blast-treated is deformed, and the desired shape may not be obtained. With respect to this, by simultaneously performing the blast treatment and the cooling, the rough region having the desired shape can be more easily obtained. In particular, when the base material contains a thermoplastic resin as the plastic, deformation by the heat build-up tends to occur, and effect of obtaining the surface having an antibacterial action by performing the cooling is remarkable.

The method of cooling is not particularly limited, and may be cooling by bringing water or a coolant into contact with the base material surface, may be cooling of the surrounding air by using dry ice or the like, or may be cooling from the back face by using a heat exchanger in a case where blast treatment is performed continuously for a base material having a small thickness, such as a film.

The blast treatment can be performed by using an ordinary blasting device. Specifically, sandblasting devices "PNEUMA-BLASTER" model SG gravity type, model FD direct pressure type, and model SC fine powder abrasive media type, available from Fuji Manufacturing Co., Ltd., can be used.

These conditions of the blast treatment can be adjusted to conditions that form the rough region having the shape described above based on the type and various physical properties of the resin composition.

The plastic molded article manufactured by performing the mechanical processing may be further molded or combined with other components. For example, a surface of a film-like or sheet-like molded article is subjected to blast treatment to form the surface into the rough region and then the molded article was formed into a bag shape or tube shape, whereby a bag-like or tube-like molded article having an inner surface provided with antibacterial action can be formed.

The combining with other components can be performed before the blast treatment or after the blast treatment. By combining the plastic molded article and other components, a desired antibacterial article can be obtained.

### Antibacterial Method

The antibacterial molded article can be used for various antibacterial method.

Specifically, by bringing the antibacterial molded article into contact with a liquid, solid, or gas containing bacteria, the bacteria contained in the liquid, solid surface, or gas which has been in contact can be killed, and the liquid, solid, or gas can be sterilized. The contact is preferably performed for the rough region of the antibacterial molded article.

The contact may be performed by known methods. For example, the contact with a liquid can be performed by methods such as soaking the antibacterial molded article into such liquid that is flowing or static, spraying or atomizing such liquid to the antibacterial molded article, or applying or dropping such liquid to the antibacterial molded article. Furthermore, the contact with a solid can be performed by methods such as allowing the antibacterial molded article that is static or slides to be in contact with or pressed toward the surface of the solid that is static or slides. Furthermore, the contact with a gas can be performed by methods such as allowing the antibacterial molded article to stand still in an atmosphere containing the gas that is flowing or static, and spraying the air to the antibacterial molded article.

### Examples

Hereinafter, specific examples of the present invention will be described together with comparative examples, but the present invention is not limited thereto.

### Experiment 1

### 1-1. Preparation of Antibacterial Article

### 1-1-1. Preparation of Base Material

As the base material to be provided with the antibacterial action, the following base material film was prepared. The size of the base material film was 15 cm × 15 cm square.

Linear low density polyethylene (LLDPE): TUS-TCS#60, available from Mitsui Chemicals Tohcello, Inc.; thickness: 53 µm

### 1-1-2. Surface Treatment

The surface of the base material film was subjected to blast treatment by using FDDSR-4 or SGF-4, which is a blasting device available from Fuji Manufacturing Co., Ltd. An abrasive media in which abrasive grains were dispersed in a base material formed from a resin (SIG080-7, available from Fuji Manufacturing Co., Ltd.) was injected on the base material film from a nozzle having a nozzle diameter of 6 mm at a processing pressure of 0.1 MPa, a spray rate of 3 kg/min, a nozzle angle from 70 to 90 degrees, and a distance between the nozzle to the target to be blasted of 150 mm. The injection of the abrasive media was performed while the base material film was cooled by providing water. The processing time of the blast treatment was changed in a range from 0.07 to 0.4 sec/cm². After the blast treatment, the base material film was washed and dried, and thus a film having fine protrusions and recesses was obtained.

The blast conditions of each sample are shown in Table 1. Samples 1-1 to 1-4 to which blast treatment was performed were used as evaluation samples, and a sample to which no blast treatment was performed was used as a comparative sample.

**[Table 1]**

| Sample No. | Base material film | Blasting device | Blast media | Processing pressure [MPa] | Spray rate [kg/min] | Processing time [sec/m²] |
|---|---|---|---|---|---|---|
| 1-1 | LLDPE | FDDSR-4 | SIG080-7 | 0. 10 | 3.0 | 0.4 |
| 1 - 2 | LLDPE | FDDSR-4 | SIG080-7 | 0.10 | 3.0 | 0.26 |
| 1 - 3 | LLDPE | FDDSR-4 | SIG080-7 | 0.10 | 3.0 | 0.13 |
| 1 - 4 | LLDPE | FDDSR-4 | SIG080-7 | 0.10 | 3.0 | 0.07 |

### 1-2. Evaluation

### 1-2-1. Surface Shape

The arithmetic average roughness Ra of the roughness curve in accordance with JIS B 0601 (2013) and the average length Rsm of the roughness curve element in accordance with JIS B 0601 (2013) of each sample were determined by the following conditions.

### Measuring Instrument

3D Laser Scanning Microscope, Vk-X250, available from Keyence Corporation.

### Measurement conditions

Used laser wavelength: 658 nm
Output: 0.95 mW
Pulse width: 1 ns
Measurement magnification: 50x
Number of measurements: 11 times

Note that, at the time of measurement of Rsm, a height (depth) that is 10% or less of the maximum height and a length that is 1% or less of the length of the calculated section (10 µm) are taken as noises and are taken as a part of convexity or concavity located before or after.

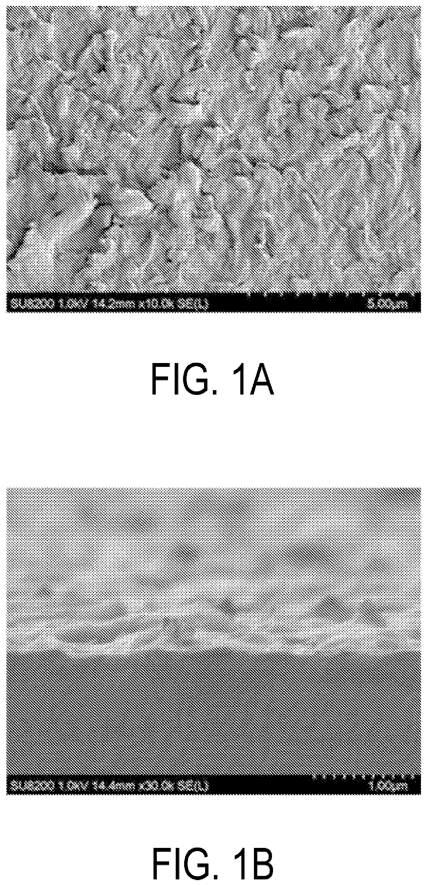

Images of the surface and cross section of each evaluation sample were taken by a scanning electron microscope (SEM). FIG. 1 shows a surface photograph and a cross section photograph of the Sample 1-1. FIG.1 A is a surface photograph with the magnification of 10000x, and FIG. 1B is a cross section photograph with the magnification of 30000x. As illustrated in FIG. 1, a large number of concavities were formed on the surface of the evaluation sample subjected to blast treatment.

### 1-2-2. Antibacterial Action

The antibacterial action against *Escherichia coli* or *Staphylococcus aureus* of each sample was evaluated. Specifically, similarly to the method described in JIS Z 2801 (2012), the following *Escherichia coli* or *Staphylococcus aureus* was inoculated and cultured for 24 hours under the following conditions. Note that, for each evaluation sample, inoculation was performed in a manner that the *Escherichia coli* or the *Staphylococcus aureus* was adhered to the rough region.

### Bacterial Strain

Escherichia coli: Escherichia coli, NBRC No. 3972
Staphylococcus aureus: Staphylococcus aureus, NBRC No. 12732

### Culture Conditions

Temperature: 35°C ± 1°C

### Measurement of Viable Bacteria Count

Used medium: standard agar medium

A viable bacteria count was measured by the method described in JIS Z 2801 (2012) immediately after the inoculation and at 24 hours after the inoculation, and the Δlog bacterial count (logarithmic value of viable bacteria count at 24 hours after comparative (unprocessed) sample - logarithmic value of viable bacteria count at 24 hours after each evaluation sample) was determined. The case where the Δlog of *Escherichia coli* or *Staphylococcus aureus* was 0.2 or greater was evaluated as exhibiting antibacterial action.

Table 2 shows the viable bacteria count (indicated by a logarithmic value) and Δlog for each evaluation sample.

**[Table 2]**

| Base material film | Sample No. | Surface roughness [µm] | | | Escherichia coli count | | Staphylococcus aureus count | | Notes |
|---|---|---|---|---|---|---|---|---|---|
| | | Ra | Rsm | Ra/Rsm | Log | ΔLog | Log | ΔLog | |
| LLDPE | 1 -1 | 0.32 | 28.5 ; | 0.0112 | 0.9 | 5.4 | -0.2 | 4.2 | Examples |
| | 1 - 2 | 0.25 | 49.5 | 0.0051 | - | - | 3.6 | 0.4 | Examples |
| | 1-3 | 0.23 | 48.6 | 0,0047 | 6.0 | 0.3 | 2.5 | 1.5 | Examples |
| | 1-4 | 0.20 | 35.7 | 0.0057 | 6.1 | 0.2 | 3.8 | 0.2 | Examples |
| | For comparison | 0.05 | 67.7 | 0,0007 | 6.3 | - | 4.0 | - | Comparative Examples |

As shown in Table 2, it was confirmed that films having the rough region having an arithmetic average roughness Ra of 0.14 µm or greater and 0.72 µm or less (Samples 1-1 to 1-4) exhibited antibacterial action.

### Experiment 2

### 2. Preparation of Base Material

### 2-1. Surface Treatment of Base Material

The six types of base material films were prepared and evaluation samples were prepared by subjecting surfaces of the base material films to blast treatment. Note that, for each base material film, comparative samples to which no surface treatment was performed were also prepared.

The used base material films are as follows. All base material films had a shape of 50 mm × 50 mm square.
Linear low density polyethylene (LLDPE): TUS-TCS#60, available from Mitsui Chemicals Tohcello, Inc.; thickness: 53 µm
Unstretched polypropylene (CPP): TORAYFAN ZK93FM, available from Toray Advanced Film Co., Ltd.; thickness: 60 µm
Polyvinylidene fluoride (PVDF): available from Kureha Corporation; thickness: 16 µm
Polyvinylidene chloride (PVDC): available from Kureha Corporation; thickness: 40 µm
Polyphenylene sulfide (PPS): available from Kureha Corporation; thickness: 50 µm
Polyethylene terephthalate (PET): TOYOBO ESTER film E5000, available from Toyobo Co., Ltd.; thickness: 75 µm

### 2-1-2. Surface Treatment

Surface treatment (blast treatment, washing, and drying) was performed in the same manner as in Experiment 1.

The blast conditions of each sample are shown in Table 3. Samples 2-1 to 2-6 to which blast treatment was performed were used as evaluation samples, and a sample to which no blast treatment was performed for each film was used as a comparative sample. Note that Sample 2-1 is identical to Sample 1-1 of Experiment 1.

**[Table 3]**

| Sample No. | Base material film | Blasting device | Blast media | Processing pressure [MPa] | Spray rate [kg/min] | Processing time [sec/m²] |
|---|---|---|---|---|---|---|
| 2 - 1 | LLDPE | FDDSR-4 | SIG080-7 | 0.10 | 3.0 | 0.4 |
| 2-2 | CPP | FDDSR-4 | SIG080-7 | 0.10 | 3.0 | 0.4 |
| 2-3 | PVDF | FDDSR-4 | SIG080-7 | 0. 10 | 3.0 | 0. 4 |
| 2-4 | PVDC | FDDSR-4 | SIG080-7 | 0.10 | 3.0 | 0. 4 |
| 2-5 | PPS | FDDSR-4 | SIG080-7 | 0.10 | 3.0 | 0.4 |
| 2-6 | PET | FDDSR-4 | SIG080-7 | 0.10 | 3.0 | 0.4 |
| 2-7 | PVDF | SGF-4 | SiC#400 | 0.10 | 1. 0 | 0.2 |
| 2-8 | PET | SGF-4 | SiC#400 | 0.10 | 1.0 | 0.2 |

### 2. Measurement of Surface Shape

Measurement of the surface shape was performed in the same manner as in Experiment 1.

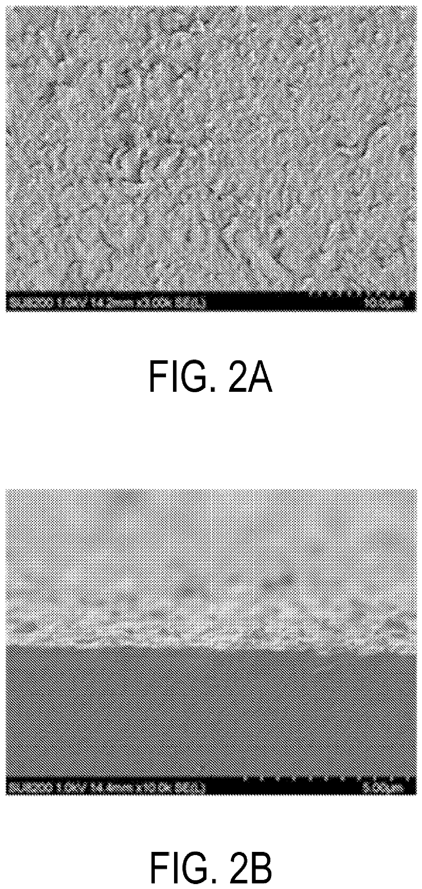

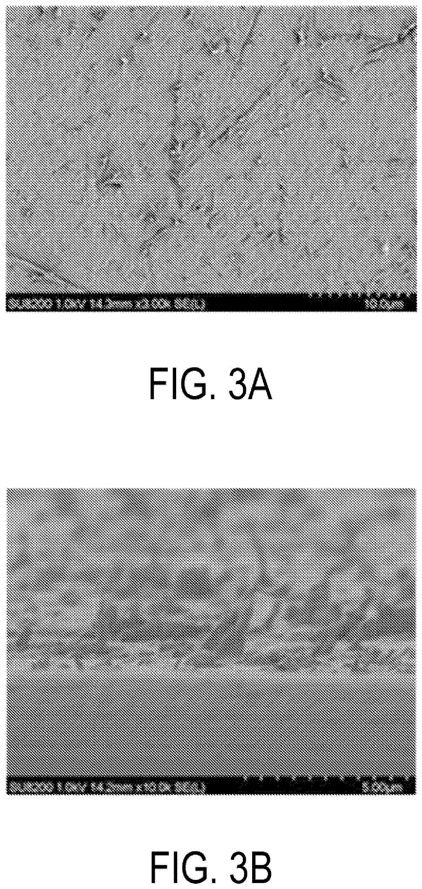

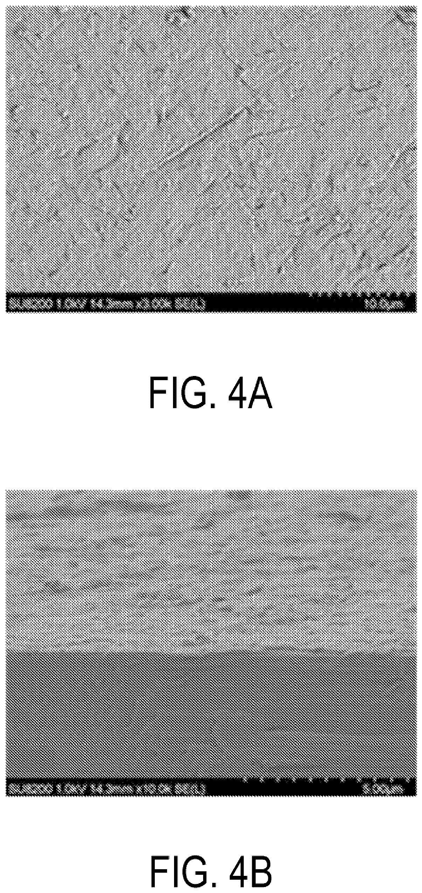

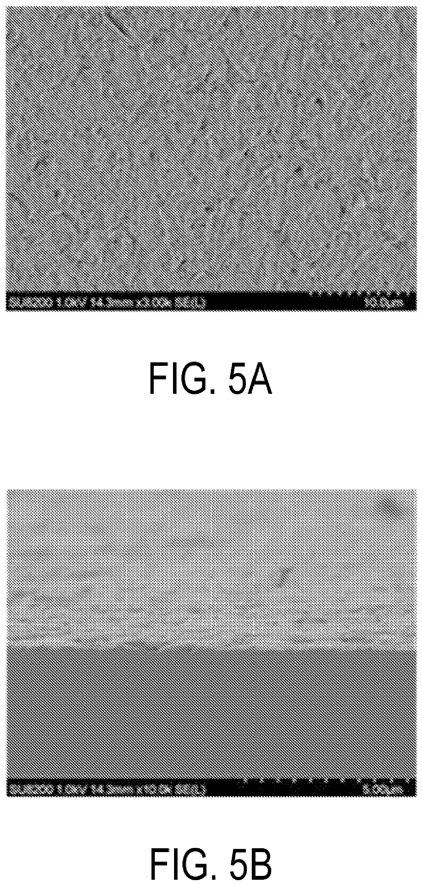

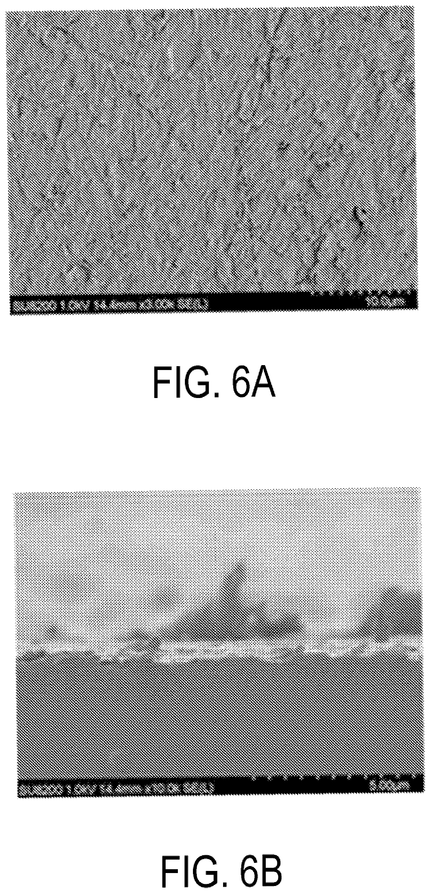

Images of the surface and cross section of each evaluation sample were taken by a scanning electron microscope (SEM). FIGS. 2 to 6 show surface photographs and cross section photographs of Samples 2-1 and 2-3 to 2-6 at the magnification of 3000x. FIG. 2A is a surface photograph of Sample 2-1 (LLDPE), and FIG. 2B is a cross section photograph of Sample 2-1 (LLDPE). FIG. 3A is a surface photograph of Sample 2-3 (PVDF), and FIG. 3B is a cross-sectional photograph of Sample 2-3 (PVDF). FIG. 4A is a surface photograph of Sample 2-4 (PVDC), and FIG. 4B is a cross-sectional photograph of Sample 2-4 (PVDC). FIG. 5A is a surface photograph of Sample 2-5 (PPS), and FIG. 5B is a cross-sectional photograph of Sample 2-5 (PPS). FIG. 6A is a surface photograph of Sample 2-6 (PET), and FIG. 6B is a cross-sectional photograph of Sample 2-6 (PET).

### 3. Antibacterial Action Evaluation

Antibacterial action evaluation was performed in the same manner as in Experiment 1.

Table 4 shows the surface roughness for each of the evaluation samples and comparative samples (arithmetic average roughness Ra and average length Rsm of profile element) and Δlog for evaluation samples. Note that, as the results for LLDPE, results obtained by Sample 1-1 and comparative sample of Experiment 1 are shown again.

**[Table 4]**

| Base materi al film | Sample No. | Surface roughness [µm] | | | Escherichia coli count | | Staphylococcus aureus count | | Notes |
|---|---|---|---|---|---|---|---|---|---|
| | | Ra | Rsm | Ra/Rsm | Log | Δlog | Log | Δlog | |
| LLDPE | 2-1 | 0.32 | 28.5 | 0.0112 | 0.2 | 5.8 | -0.2 | 4.2 | Examples |
| | For comparison | 0.05 | 67.7 | 0.0007 | 6.0 | - | 4.0 | - | Comparative Examples |
| CPP | 2-2 | 0.30 | 24.1 | 0.0124 | 1.4 | 4.7 | 1.1 | 2.9 | Examples |
| | For comparison | 0.07 | 57.5 | 0.0012 | 6.1 | - | 4.0 | - | Comparative Examples |
| PVDF | 2-3 | 0.18 | 35.8 | 0.0050 | 5.2 | 1.1 | 2.8 | 1.4 | Examples |
| | For comparison | 0.06 | 30.7 | 0.0019 | 6.3 | - | 42 | - | Comparative Examples |
| PVDC | 2-4 | 0.25 | 27.1 | 0.0092 | 2.4 | 3.7 | -0.2 | 4.3 | Examples |
| | For comparison | 0.13 | 36.3 | 0.0034 | 6.1 | - | 4.1 | - | Comparative Examples |
| PPS | 2-5 | 0.23 | 32.5 | 0.0072 | 1.5 | 4.7 | 0.7 | 3.3 | Examples |
| | For comparison | 0.01 | 44.5 | 0.0003 | 6.2 | - | 4.0 | - | Comparative Examples |
| PET | 2-6 | 0.24 | 31.0 | 0.0078 | 5.2 | 1.1 | -0.2 | 4.2 | Examples |
| | For comparison | 0.06 | 64.5 | 0.0009 | 6.3 | - | 4.0 | - | Comparative Examples |
| PVDF | 2 - 7 | 0.73 | 25.8 | 0.0282 | 6.2 | 0.1 | 4.2 | 0.0 | Comparative Examples |
| | For comparison | 0.06 | 30.7 | 0.0019 | 6.3 | - | 4.2 | - | Comparative Examples |
| PET | 2 - 8 | 1.11 | 30.8 | 0.0359 | 6.3 | 0.0 | 3.9 | 0.1 | Comparative Examples |
| | For comparison | 0.06 | 64.5 | 0.0009 | 6.3 | - | 4.0 | - | Comparative Examples |

As shown in Table 4, it was confirmed that evaluation samples having the rough region having an arithmetic average roughness Ra of 0.14 µm or greater and 0.72 µm or less exhibited high antibacterial action. Furthermore, the LLDPE having the rough region exhibited especially high antibacterial action.

The present application claims priority to the Japanese Patent Application No. 2019-184043 filed on October 4, 2019, and the contents of the claims, the specification, and the drawings of this application are incorporated into the present application.

### Industrial Applicability

The antibacterial molded article of an embodiment of the present invention can be used in various use in which sterilizing and antibacterial actions are desired.

## Claims

1. An antibacterial molded article comprising:
a plastic molded article,
the plastic molded article having a rough region having an arithmetic average roughness Ra of a roughness curve in accordance with JIS B 0601 (2013) of 0.14 µm or greater and 0.72 µm or less provided on a surface with which bacteria can come into contact.

2. The antibacterial molded article according to claim 1, wherein the rough region has an average length Rsm of a profile element in accordance with JIS B 0601 (2013) of 1.0 µm or greater and 50.0 µm or less.

3. The antibacterial molded article according to claim 1 or 2, wherein the rough region is a region in which many concavities are formed.

4. The antibacterial molded article according to any one of claims 1 to 3, wherein the rough region has a ratio (Ra/Rsm) of an arithmetic average roughness Ra of a roughness curve in accordance with JIS B 0601 (2013) to an average length Rsm of a profile element in accordance with JIS B 0601 (2013) of 0.004 or greater and 0.720 or less.

5. The antibacterial molded article according to any one of claims 1 to 4, wherein the plastic molded article is a molded article containing at least one resin selected from the group consisting of polyolefin-based resins, polytetrafluoroethylene (PTFE), perfluoroalkoxy alkane (PFA), perfluoroethylene propene copolymers (FEP), polyvinylidene fluoride (PVDF), polyvinylidene chloride (PVDC), polyamide (PA), polyimide (PI), polyphenylene sulfide (PPS), polyether ether ketone (PEEK), polyacetal (POM), acrylonitrile-butadiene-styrene copolymer (ABS) resins, polycarbonate (PC), polyethylene terephthalate (PET), polyurethane, ethylene-vinyl alcohol copolymers (EVOH), polyvinyl chloride (PVC), acrylic polymers, ethylene-vinyl acetate copolymers (EVA), polylactic acid (PLA), polycaprolactone (PCL), and polyglycolic acid (PGA).

6. The antibacterial molded article according to any one of claims 1 to 5, wherein the plastic molded article is a molded article containing at least one resin selected from the group consisting of polyethylene, polypropylene, polyvinylidene chloride, polyethylene terephthalate, and polyphenylene sulfide.

7. The antibacterial molded article according to any one of claims 1 to 6, wherein the antibacterial molded article is a packaging, equipment for a building, a home appliance, an electronic device or peripheral device thereof, a component for automobiles, various coatings, medical equipment, an article for agriculture, stationery or body outfit.

8. The antibacterial molded article according to any one of claims 1 to 7, wherein the antibacterial molded article is forceps, a syringe, a stent, an artificial blood vessel, a catheter, a wound dressing, a scaffolding for regenerative therapy, an antiadhesive material, or a pacemaker.

9. A method for manufacturing an antibacterial molded article, the method comprising:
preparing a base material containing a plastic on a surface, and
performing a blast treatment at least on the surface containing the plastic of the base material.

10. The method for manufacturing an antibacterial molded article according to claim 9, wherein
the performing the blast treatment is
a process of allowing an abrasive media energized by a compressed fluid to collide with the surface containing the plastic of the base material while the base material is cooled.

11. The method for manufacturing an antibacterial molded article according to claim 10, wherein the abrasive media contains an abrasive grain and an elastic body.
